# EUROPEAN PATENT SPECIFICATION

(11) **EP 3 711 804 B1**
(45) Date of publication and mention of the grant of the patent: **18.06.2025**
(21) Application number: 17929072.1
(22) Date of filing: 20.10.2017
(51) Int. Cl.: A61M 16/01, A61M 16/08, G01N 33/00, A61M 16/10, A61M 16/20, A61M 16/22

(54) **ANESTHESIA MACHINE AND CALIBRATION METHOD**
ANÄSTHESIEMASCHINE UND KALIBRIERUNGSVERFAHREN
MACHINE D'ANESTHÉSIE ET PROCÉDÉ D'ÉTALONNAGE

(43) Date of publication of application: 23.09.2020
(73) Proprietor: Shenzhen Mindray Bio-Medical Electronics Co., Ltd, Guangdong 518057 (CN); Shenzhen Mindray Scientific Co., Ltd., Shenzhen, Guangdong 518000 (CN)
(72) Inventor: WU, Xuetao, Shenzhen, Guangdong 518057 (CN); CHEN, Peitao, Shenzhen, Guangdong 518057 (CN)
(74) Representative: KIPA AB
(86) International application number: PCT/CN2017/107102
(87) International publication number: WO 2019/075747

(56) References cited:
- EP-A1- 2 474 333
- WO-A1-2008/000299
- WO-A1-2009/062550
- CN-A- 101 288 791
- CN-A- 101 468 221
- CN-A- 102 580 213
- CN-A- 102 721 691
- CN-A- 103 893 879
- CN-A- 104 215 677
- CN-A- 105 476 637
- CN-A- 105 517 612
- CN-A- 105 597 210
- CN-U- 203 786 078
- US-A- 5 806 513
- US-A- 5 806 513
- US-A1- 2002 148 471
- US-A1- 2011 114 094
- US-A1- 2014 326 046
- US-A1- 2015 233 879
- US-A1- 2015 297 857
- US-A1- 2015 374 948
- US-B1- 6 520 180
- US-B2- 7 788 963
- US-B2- 8 887 724

## Description

### Technical Field

The present disclosure relates to the technical field of anesthesia equipment, and in particular to an anesthesia machine, an oxygen sensor calibration system and a calibration method thereof.

### Background

During the ventilation of anesthesia machines, different oxygen concentrations will be given according to the individual conditions of patients. In order to ensure that the oxygen concentration inhaled by a patient is within a set range, the anesthesia machine will generally be equipped with an oxygen sensor in a breathing circuit to monitor the oxygen concentration of gas in real time.

Oxygen batteries commonly used in the anesthesia machines are divided into chemical oxygen batteries and paramagnetic oxygen batteries. The former realizes the measurement of oxygen concentration by means of the mechanism that oxygen molecules react with a specific chemical substance in the oxygen sensor to generate a current. Different currents will be generated when different concentrations of oxygen enter the oxygen sensor. Before use, the oxygen sensor needs to be calibrated using a gas with known oxygen concentrations, usually at two points (such as oxygen concentrations of 21% and 100%), so as to obtain a corresponding linear relationship between the oxygen concentration and the current. When measuring the oxygen concentration in actual work, a reverse solution is carried out according to the measured current and the corresponding function relationship between the oxygen concentration and the current to obtain a current value of the oxygen concentration. During the operation of the chemical oxygen sensor, as the measurement time passes, the corresponding relationship between the oxygen concentration and the current will drift due to the change in the form of the chemical substance. In order to ensure the accuracy and reliability of measurement of the oxygen concentration, the oxygen sensor needs to be calibrated regularly within the service life of the oxygen sensor. The paramagnetic oxygen sensor is based on the paramagnetic characteristics of oxygen. A typical measurement method consists in that when a gas to be measured enters the paramagnetic oxygen sensor, the gas will be sucked into a magnetic field and impact on the internal physical structure thereof, which causes the internal physical structure to generate a deflection moment to obtain a linear relationship between the oxygen concentration and the moment (current). During measuring the oxygen concentration in actual work, the reverse solution is carried out according to the actually measured magnitude of the current and the corresponding function relationship between the oxygen concentration and the current to obtain the current value of the oxygen concentration. The paramagnetic oxygen sensor carries out the measurement by means of purely physical principles. Theoretically, there is no limit on the service life, however, usually due to factors such as movement and impact, the internal structure may be changed, causing measurement deviations. Therefore, the paramagnetic oxygen sensor also needs to be calibrated usually at one point (e.g. oxygen concentration of 100%) according to actual requirements.

At present, most of the anesthesia machines on the market use a chemical oxygen sensor. When the calibration is performed at the oxygen concentration points of 21% and 100%, it is usually necessary to remove the oxygen sensor and place it in the air for a period of time, such as 1 to 3 minutes. Then, calibration operations are performed in a calibration procedure to acquire the current value of the current output by the oxygen sensor, and the oxygen sensor is then mounted back to the anesthesia machine. A fresh gas (pure oxygen) is adjusted to flush the circuit for a period of time, usually 1 to 3 minutes, and the calibration operations are then performed in the calibration procedure to acquire the current value of the current output by the oxygen sensor. The earlier and later current values respectively correspond to the points where the oxygen concentrations are 21% and 100%, so as to obtain the linear relationship between the oxygen concentration and the current. The current calibration operations require manual intervention, and the operation procedure is complicated and time-consuming.

D1 (EP2474333A1) is about selectively operating a fresh gas valve (52 of D1) between two positions to provide mechanical support to a patient via alternative flow paths (54, 56 of D1, see Fig. 1 of D1) in relation to an inspiratory valve (30 of D1). The purpose is to provide a rapid gas exchange in the inspiratory limb, see [0050] of D1.

D2 (US6520180B1) discloses a system to calibrate an oxygen sensor to overcome the effects of pressure change during a breath. Pressure variations occur in the breathing gas line due to the interplay of inspiration and expiration, and they affect the accuracy of the oxygen concentration measurement. Even though it would be possible to calculate correction values for the oxygen concentration by the pressure measurement, which is performed in the vicinity of the oxygen sensor, complicated evaluating logarithms are needed for this, because both the course of the pressure over time and the course of the oxygen concentration over time must be evaluated simultaneously. Correspondingly corrected measured values for the oxygen concentration are therefore available only after a certain time and therefore they also contain a high inaccuracy. If the oxygen sensor is used in the control circuit as an actual value transducer for the oxygen concentration to be set, delays and inaccuracies in the determination of the actual value are not acceptable, because these would compromise the control.

D3 (US2014326046A1) is an application of the same Applicant. It discloses a gas treatment device. Gas treatment devices in this disclosure can provide guidance in a gas flow direction through gas circuits in an integrated gas circuit board. In this case, the gas to be monitored can be in natural circulation and/or be sampled in real time, and at least two kinds of gas treatments can be performed on the gas thereafter. For instance, some gas treatments such as concentration monitoring, mechanics parameter monitoring and/or negative pressure processing may be performed on the measured gas as required. Through such gas treatment devices, a simplified gas circuit structure can be achieved, and the gas circuits can be conveniently connected by effectively reducing the usage of hose for gas circuit connection.

D4 (US7788963B2) discloses a system adapted to calibrate a determination of information related to one or more gaseous analytes in a body of gas being delivered to an objective by a gas source, the system comprising: a partial pressure sensor that generates an output signal related to the partial pressure of the one or more gaseous analytes in the body of gas; a total pressure monitor that determines the total pressure of the body of gas; a partial pressure module that determines the partial pressure of the one or more gaseous analytes in the body of gas according to a partial pressure function, wherein the partial pressure function describes the partial pressure of the one or more gaseous analytes in the body of gas as a function of the output signal generated by the partial pressure sensor; and a calibration module that calibrates the partial pressure module by determining the partial pressure function, wherein the calibration module determines the partial pressure function based on (i) a plurality of samples of the output signal generated by the partial pressure sensor at a plurality of points in time during a calibration time period, (ii) a plurality of determinations of the total pressure of the body of gas made at substantially the same points in time at which the plurality of samples of the output signal were generated, and (iii) a target concentration of the one or more analytes in the body of gas during the calibration time period, wherein the calibration time period comprises a time period during which the body of gas is being delivered to the objective while the gas source operates to keep the concentration of the one or more analytes in the body of gas substantially equal to the target concentration as the total pressure of the body of gas changes.

D5 (US2011114094A1) discloses: All galvanic oxygen sensors must be calibrated to ensure accurate readings. If a sensor falls out of proper calibration the electronic control system of a CCR will misinterpret the readings. A calibration process typically involves exposing the sensors to one or more known gas mixtures at a known ambient pressure, and deriving calibration constants to the electronic logic that interprets the sensor readings. Calibration is typically conducted manually or semi-automatically prior to the dive, but is sometimes only done periodically. Calibration constants can be recorded incorrectly if the calibration gas mixture deviates from expected (e.g., if the calibration process assumes a mixture of 100 percent oxygen when a contaminated calibration gas is actually only 80 percent oxygen), if the ambient pressure is not properly taken into account, if the sensor fails in certain ways as described below, and/or if the user performs the calibration process incorrectly. Attempts to mitigate these problems have included automated calibration routines as part of the standard pre-dive process, incorporation of ambient pressure sensors into the calibration process, and testing against threshold values intended to detect calibration errors.

Galvanic oxygen sensors eventually fail either through exhaustion of their chemical reaction or other age related degradation of active sensing elements and/or from a host of other environmental and user-caused effects (e.g. abuse, improper use). In many cases, a sensor will simply fail to generate sufficient output voltage at the time of calibration, and will be identified. In other cases, however, a sensor can perform normally up to a certain point, but deviate significantly from linearity in output voltage once the oxygen concentration exceeds a certain value. For example, a sensor could perform normally up to an oxygen concentration of 1.1 bar partial pressure, but then fail to produce a correspondingly higher output voltage at higher oxygen concentrations. Because the calibration process of most CCR systems uses 100 percent oxygen at ambient pressure (i.e, 1 bar partial pressure) in a pre-dive calibration, the calibration process may appear to complete correctly, but the system may not be able to properly interpret readings when the sensor is exposed to oxygen partial pressures above 1 bar.

D6 (US2015297857A1) discloses an anesthetic breathing apparatus for providing or regulating a plurality of anesthetic agents in a breathing circuit, e.g. without impairing patient safety.

D7 (US2002148471A1) provides a radiative artificial respiration system which carries out a low-flow anesthesia method while minimizing increases in the temperature of the carbon dioxide absorbent, thus reducing evaporation of moisture from the carbon dioxide absorbent and the amount of condensation formed in the anesthetic circuit, but without increasing the amount of decomposed compounds produced by the reaction between a volatile anesthetic and the carbon dioxide absorbent. D7 also provides a new artificial respirator that can inexpensively and easily solve the above problems with low-flow anesthesia. This will serve as the basis for the diffusion of low-flow anesthesia, which reduces costs, prevents operation-room employees from being exposed to gas, and reduces the amount of anesthetic gas exhausted to the atmosphere.

D8 (US5806513A) provides an anesthesia delivery system control scheme which achieves satisfactory automatic control of gas and vapor concentrations at low or minimal fresh gas flows and throughout variations in the rate of flow of fresh gas. D8 also provides an anesthesia delivery system which conserves anesthesia delivery system gases, anesthetic agent and patient heat and humidity, an anesthesia delivery system that permits clinicians to set a minimum fresh gas flow to the breathing circuit, and an anesthesia delivery system which performs a circuit fill operation without altering the gas and anesthetic agent concentrations inspired by the patient.

D9 (WO2009062550A1) discloses an anesthetic breathing apparatus which comprises, a first gas analyzer (200) arranged to provide gas measurement data related to a first sample point (190) to a control unit (65) that is arranged in a servo or feedback control system configured to adjust delivery of a vaporized anesthetic agent from an anesthetic vaporizer (22) to a delivery point (19) in an inspiratory branch of a breathing circuit (7) of said anesthetic breathing apparatus, wherein the first sampling point (190) is arranged in the breathing circuit (7) downstream and close to the delivery point (19); a second gas analyzer (210) arranged to be operated in a first mode of operation to provide gas measurement data for patient monitoring, and arranged to be operated in a second mode of operation to provide gas measurement data to the control unit (65); and a switch over unit (240) fluidly connected to the second gas analyzer (210), and arranged to switch fluid communication of the second gas analyzer (210) between the first sample point (190) for the second mode of operation and a second sample point (290) for the first mode of operation, wherein the second sample point (290) is arranged in a patient tubing (2) close to a Y-piece (4) at a patient (1) connected to the breathing circuit (7).

D10 (CN105597210A) discloses an anesthesia machine which is compatible with an ascending type bellows and a descending type bellows.

### Summary

In view of this, it is necessary to provide an oxygen sensor calibration system in response to the problem of the current anesthesia machine requiring manual intervention during automatic calibration of the oxygen sensor, an anesthesia machine containing the above-mentioned oxygen sensor calibration system is further provided, and an calibration method applied to the above-mentioned oxygen sensor calibration system is provided.

According to the invention, there is provided an anesthesia machine according to claim 1, an anesthesia machine according to claim 2, and a method according to claim 8. The dependent claims define embodiments of the invention.

References to "embodiments" throughout the description which are not under the scope of the appended claims merely represents possible exemplary executions and are therefore not part of the present invention, although they may have features that may be present in embodiments of the invention as claimed.

With the use of the above technical solutions, the present disclosure has the beneficial effects as follows:
The anesthesia machine, the oxygen sensor calibration system and the calibration method thereof of the present disclosure may realize automatic calibration of an oxygen sensor without manual intervention, ensure the reliability the operation of the oxygen sensor, and enable the anesthesia machine to operate normally.

### Brief Description of the Drawings

Fig. 1 is a schematic diagram of an oxygen sensor calibration system of an anesthesia machine according to an embodiment of the invention as claimed;
Fig. 2 is a schematic diagram of an implementation of an oxygen sensor calibration system of an anesthesia machine according to another embodiment of the invention as claimed;
Fig. 3 is a schematic diagram of another implementation of the oxygen sensor calibration system shown in Fig. 2, which is part of an anesthesia machine according to an embodiment of the invention as claimed; and
Fig. 4 is a schematic diagram of a gas path of an anesthesia machine of another embodiment of the present disclosure.

In the figures:
100 - Oxygen sensor calibration system;
110 - Breathing circuit; 111 - Inspiratory branch; 112 - Expiratory branch; 113 - Absorption tank branch; 114 - Inspiratory one-way valve; 115 - Expiratory one-way valve; 116 - Connection pipeline; 117 - CO₂ absorption tank;
120 - Oxygen sensor;
130 - Bypass branch;
140 - Switch component;
200 - Anesthetic supply device;
300 - Expiratory device;
310 - Expiratory pipeline;
320 - Expiratory valve;
330 - Adjustment valve;
340 - Adjustment pipeline.

### Detailed Description

To make the objectives, technical solutions, and advantages of the present disclosure more apparent, the anesthesia machine, the oxygen sensor calibration system and the calibration method thereof of the present disclosure are described below in further detail by way of embodiments and with reference to the accompanying drawings. It should be understood that the particular embodiments described herein are merely intended to explain the present disclosure and is not intended to define the present disclosure.

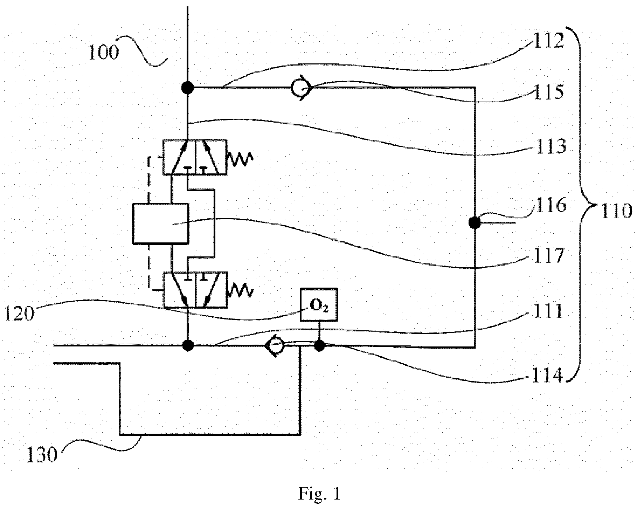

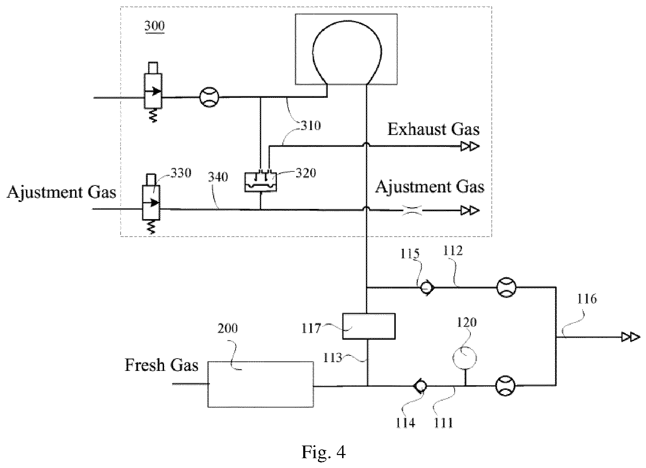

Referring to Figs. 1 to 3, the present disclosure provides an oxygen sensor calibration system 100. The oxygen sensor calibration system 100 includes a breathing circuit 110, an oxygen sensor 120, and a calibration management unit. The oxygen sensor calibration system 100 may be used in an anesthesia machine shown in Fig. 4. The oxygen sensor calibration system 100 is applied in the anesthesia machine and used to calibrate the oxygen sensor 120 of the anesthesia machine. In this way, the oxygen sensor 120 may reliably detect the oxygen concentration in the gas in the breathing circuit 110 when the anesthesia machine is in use, such that the oxygen concentration in the gas supplied to a patient may meet actual demands and the use safety of the patient is ensured. If the oxygen sensor 120 needs to be calibrated, a calibration gas flowing through the oxygen sensor 120 is used to perform the oxygen concentration calibration. The oxygen sensor 120 here refers to a medical oxygen sensor. Of course, in other implementations of the present disclosure, not according to the invention as claimed, the oxygen sensor calibration system 100 of the present disclosure may also be used in the oxygen concentration calibration of the oxygen sensor 120 in other apparatuses, such as a ventilator.

In the present disclosure, the breathing circuit 110 includes an inspiratory branch 111, an expiratory branch 112, an absorption tank branch 113, an inspiratory one-way valve 114, a connection line 116, and an expiratory one-way valve 115. The inspiratory branch 111 communicates with the expiratory branch 112 through the connection pipeline 116. The inspiratory one-way valve 114 is arranged in the inspiratory branch 111, and the expiratory one-way valve 115 is arranged in the expiratory branch 112. One end of the absorption tank branch 113 communicates with the inspiratory branch 111, and the one end of the absorption tank branch 113 is located at a front end of the inspiratory one-way valve 114. The other end of the absorption tank branch 113 communicates with the expiratory branch 112 and is located at a rear end of the expiratory one-way valve 115. The oxygen sensor 120 is connected to the inspiratory branch 111, with a joint being located at a rear end of the inspiratory one-way valve 114.

During normal use of the anesthesia machine, a first end of the breathing circuit 110 communicates with an anesthetic supply device 200, a second end of the breathing circuit 110 communicates with an exhaust gas discharge device, and the breathing circuit 110 also communicates with a breathing end of the patient. The first end here refers to a gas inlet end of the breathing circuit 110, and the second end refers to a gas outlet end of the breathing circuit 110. The anesthetic supply device 200 supplies an inspiratory gas containing an anesthetic to the breathing circuit 110 and delivers the gas to the patient. The exhaled gas containing the anesthetic exhaled from the patient is purified in the circuit by a CO₂ absorption tank 117 and is then reused, and the remaining gas is processed by the exhaust gas discharge device. Moreover, the exhaust gas discharge device may be either an exhaust gas discharge device or an exhaust gas recovery device, or may further be another device capable of processing exhaust gas.

Specifically, a gas inlet end of the inspiratory branch 111 is capable of communicating with the anesthetic supply device 200 of the anesthesia machine. The connection pipeline 116 is used to connect a gas outlet end of the inspiratory branch 111, a gas inlet end of the expiratory branch 112 and the breathing end of the patient. A gas outlet end of the expiratory branch 112 communicates with the exhaust gas discharge device. Here, the gas inlet end of the inspiratory branch 111 coincides with the gas inlet end of the breathing circuit 110 and they are the same end, and the gas outlet end of the expiratory branch 112 coincides with the gas outlet end of the breathing circuit 110 and they are the same end. The inspiratory gas containing the anesthetic delivered by the anesthetic supply device enters the inspiratory branch 111, passes through the inspiratory branch 111 and then enters the breathing end of the patient through the connection pipeline 116 to supply the patient with the anesthetic. The exhaled gas from the patient enters the connection pipeline 116 via the breathing end, and enters the expiratory branch 112 through the connection pipeline 116. In an inhalation phase, the exhaled gas passes through the CO₂ absorption tank 117, and is reused after CO₂ is absorbed, and the remaining gas is discharged from the exhaust gas discharge device via an expiratory valve. Moreover, the connection pipeline 116 includes a Y-type pipe or a communicating pipe for connecting the inspiratory branch 111 and the expiratory branch 112, and ends of the Y-type pipe are respectively connected to the inspiratory branch 111, the expiratory branch 112 and the patient. The Y-type pipe in capable of facilitating the inspiratory gas to enter the breathing end of the patient through the inspiratory branch 111, and also allows the exhaled gas from the patient to enter the expiratory branch 112. Also, the inspiratory one-way valve 114 is arranged in the inspiratory branch 111, which may prevent the inspiratory gas flowing through the inspiratory one-way valve 114 from returning, so that the inspiratory gas flows in a single direction. The expiratory one-way valve 115 is arranged in the expiratory branch 112, which may prevent the exhaled gas flowing through the expiratory one-way valve 115 from returning, so that the exhaled gas flows in a single direction. The inspiratory one-way valve 114 is located downstream of the joint between the inspiratory branch 111 and the absorption tank branch 113, that is, the inspiratory gas first passes through the joint between the inspiratory branch 111 and the absorption tank branch 113 in the inspiratory branch 111 and then flows through the inspiratory one-way valve 114. The expiratory one-way valve 115 is located upstream of the joint between the expiratory branch 112 and the absorption tank branch 113, that is, the exhaled gas first passes through the expiratory one-way valve 115 in the expiratory branch 112 and then flows through the joint between the expiratory branch 112 and the absorption tank branch 113.

The oxygen sensor 120 is used to detect whether the oxygen concentration in the inspiratory gas delivered by the anesthesia machine reaches a standard, specifically: the oxygen sensor 120 is capable of detecting the oxygen concentration in the inspiratory gas delivered to the patient through the inspiratory branch **111** to ensure that the oxygen concentration in the inspiratory gas containing the anesthetic supplied to the patient may meet the demands and ensure the safety during use. If the oxygen concentration in the inspiratory gas containing the anesthetic is lower or higher than a pre-set oxygen concentration for the anesthesia machine, it may cause potential hazards to the safety of the patient. After the oxygen sensor 120 has been used for a period of time, there may be a certain deviation between the oxygen concentration detected by the oxygen sensor 120 and the actual oxygen concentration. Therefore, the oxygen sensor 120 needs to be calibrated regularly or as required to ensure that the oxygen sensor 120 may accurately detect the oxygen concentration in the inspiratory gas, to ensure the reliability of detection of the oxygen concentration, and in turn to ensure the reliable operation of the anesthesia machine.

Moreover, the oxygen concentration calibration of the oxygen sensor 120 is achieved by using a calibration gas, and the calibration management unit is used to control the flow of the calibration gas. If the oxygen sensor 120 needs to be calibrated, the calibration management unit controls the calibration gas to enter the inspiratory branch **111** and pass through the oxygen sensor 120, the connection pipeline 116, and the expiratory branch 112. The calibration management unit performs the oxygen concentration calibration according to the calibration gas flowing through the oxygen sensor 120. Specifically, the calibration management unit collects the current output by the oxygen sensor 120 that corresponds to the oxygen content in the calibration gas to obtain a linear function relationship between the oxygen concentration and the output current. The calibration management unit may further store the obtained linear function relation for the oxygen concentration in a memory of the anesthesia machine. During a subsequent oxygen concentration measurement, a control unit of the anesthesia machine may obtain the oxygen concentration of the inspected gas by means of the reverse calculation from the value of the current output from the oxygen sensor 120 according to the linear function relationship for the oxygen concentration stored in the memory. In a specific embodiment, the control unit of the anesthesia machine and the calibration management unit may be either two different components or the same component, for example a board integrated with software algorithms and a controller.

Specifically, if the oxygen sensor 120 is a chemical oxygen sensor, the calibration management unit may calibrate the oxygen sensor 120 using a calibration gas having at least two different oxygen concentrations. In this way, the accuracy of the concentration calibration of the oxygen sensor 120 may be ensured and the safety coefficient during use is improved. In an implementation of the present disclosure, a calibration gas having an oxygen concentration of 21% and a calibration gas having an oxygen concentration of 100% are respectively introduced into the breathing circuit 110. When the oxygen sensor 120 is being calibrated, firstly, the calibration gas having the oxygen concentration of 21% is introduced into the inspiratory branch 111 at a flow rate of 5 L/min for 1 minute to stabilize the value of the current output by the oxygen sensor 120, and the calibration management unit collects and stores the current value of the current output by the oxygen sensor 120; secondly, the calibration gas having the oxygen concentration of 100% is introduced into the inspiratory branch 111 at a flow rate of 5 L/min for 1 minute to stabilize the value of the current output by the oxygen sensor 120, the calibration management unit samples the current value of the current output by the oxygen sensor 120 and stores same in the memory. The calibration management unit obtains a linear function relation for the oxygen concentration according to the corresponding relationship between the values of the current and the values of the oxygen concentrations at two calibration points, and stores same in the memory, completing the calibration operation of the oxygen sensor 120. Of course, the oxygen concentrations of the calibration gas introduced into the inspiratory branch 111 may also be selected to be any other two controllable oxygen concentrations, such as 30% and 90%, etc. Of course, in addition to the calibration gases having the above-mentioned oxygen concentrations of 21% and 100% introduced into the breathing circuit 110, the calibration gas having one of more of the oxygen concentrations of 30%, 40% and 90% may also be introduced. Furthermore, if the requirements for calibration effects are not too high, the calibration may also be performed by using only the calibration gas having one oxygen concentration.

Of course, in another implementation of the present disclosure, the oxygen sensor 120 is a paramagnetic oxygen sensor, and the calibration management unit calibrates the oxygen sensor 120 with the calibration gas having at least one oxygen concentration. The paramagnetic oxygen sensor may be calibrated with the calibration gas having only one oxygen concentration, or may also be calibrated with the calibration gas having two or more oxygen concentrations. The calibration gas having the oxygen concentration of 21% or the calibration gas having the oxygen concentration of 100% is introduced into the inspiratory branch 111. When the oxygen sensor 120 is being calibrated, the calibration gas having the oxygen concentration of 21% is introduced into the breathing circuit 110 at the flow rate of 5 L/min for 1 minute to stabilize the current output by the oxygen sensor 120, the calibration management unit collects and stores the current value of the current output by the oxygen sensor 120, and the calibration management unit samples and stores the current value of the current output by the oxygen sensor 120 in the memory. Alternatively, the calibration gas having the oxygen concentration of 100% is introduced into the breathing circuit 110 at the flow rate of 5 L/min for 1 minute to stabilize the current output by the oxygen sensor 120, the calibration management unit collects and stores the current value of the current output by the oxygen sensor 120, and the calibration management unit samples and stores the current value of the current output by the oxygen sensor 120 in the memory. The control unit draws a linear function relation according to the corresponding relationship between the value of the current and the oxygen concentration value at the calibration point and stores same in the memory, completing the calibration operation of the oxygen sensor 120. Of course, the calibration gas having the oxygen concentration of 21% and the calibration gas having the oxygen concentration of 100% may also be respectively introduced into the inspiratory branch 111. Of course, the oxygen concentration of the calibration gas introduced into the inspiratory branch 110 may also be selected to be any one or two other controllable oxygen concentrations, such as 30% and 90%. Of course, in addition to the calibration gases having the above-mentioned oxygen concentrations of 21% and 100% introduced into the breathing circuit 110, the calibration gas having one of more of the oxygen concentrations of 30%, 40% and 90% may also be introduced.

In the above calibration operation, the flow rate and the period of time of the calibration gas introduced into the inspiratory branch 111 may also be set according to the specific structure and volume of the breathing circuit, for example, flow rate of 5 L/min for 3 min, flow rate of 8 L/min for 2 min, flow rate of 10 L/min for 1 min, etc. The relationship between the flow rate and the period of time is aimed to completely replace and stabilize the types of gases in a measurement area of the oxygen sensor.

During the calibration of the oxygen sensor 120, the anesthetic supply device 200 is required to be turned off, such that the calibration gas provided by the anesthesia machine enters the inspiratory branch 111, and the calibration gas is input into the inspiratory branch 111. The calibration management unit controls the calibration gas to enter the inspiratory branch 111 and flow out through the oxygen sensor 120, the connection pipeline 116 and the expiratory branch 112. The calibration management unit performs the oxygen concentration calibration according to the calibration gas flowing through the oxygen sensor 120. When the calibration gas flows through the oxygen sensor 120, the oxygen sensor 120 may output a corresponding value of the current according to the value of the actual oxygen concentration in the calibration gas, and the calibration management unit stores the obtained function relation between the values of the oxygen concentration and the current in the memory to realize the calibration operation of the oxygen sensor 120.

The oxygen sensor calibration system 100 of the present disclosure enables the calibration gas to flow through the rear end of the inspiratory one-way valve 114 in the inspiratory branch 111, pass through the connection pipeline 116 and then flow out through the expiratory branch 112. During the calibration of the oxygen sensor 120, the calibration management unit controls the calibration gas to directly enter the expiratory branch 112 through the inspiratory branch 111, without disconnecting the connection pipeline 116. Since the oxygen sensor 120 is connected to the inspiratory branch 111 at the rear end of the inspiratory one-way valve 114, when the calibration gas flows in the inspiratory branch 111, replacement with the calibration gas in the inspiratory branch 111 may occur in the measurement area of the oxygen sensor 120, such that automatic calibration of the oxygen sensor 120 is realized to ensure the reliability of the operation of the oxygen sensor 120 and to enable the anesthesia machine to operate normally. Several ways in which the calibration gas enters the connection pipeline 116 via the rear end of the inspiratory one-way valve 114 are described in detail as follows.

Referring to Fig. 1, in an embodiment of the present disclosure, the oxygen sensor calibration system 100 further includes a bypass branch 130, and the bypass branch 130 and the inspiratory branch 111 are connected between the inspiratory one-way valve 114 and the oxygen sensor 120. The calibration management unit controls the calibration gas to enter the inspiratory branch 111 through the bypass branch 130 during the calibration. That is to say, one bypass branch 130 is separately provided, and the bypass branch 130 is directly introduced into the inspiratory branch 111 at the rear end of the inspiratory one-way valve 114 and is located before the oxygen sensor 120, namely the calibration gas enters the inspiratory branch 111 through the bypass branch 130 and then flows through the oxygen sensor 120. Moreover, thanks to the effect of the inspiratory one-way valve 114, the calibration gas may only flow along the inspiratory branch 111 through the oxygen sensor 120 and the connection pipeline 116 into the expiratory branch 112, which may prevent the calibration gas from entering the absorption tank branch 113. When the oxygen sensor 120 is being calibrated, the calibration management unit controls the calibration gas to enter the inspiratory branch 111 through the bypass branch 130, and flow through the oxygen sensor 120 and the connection pipeline 116 into the expiratory branch 112, such that the automatic calibration of the oxygen sensor 120 is realized to ensure the reliability of the operation of the oxygen sensor 120 and to enable the anesthesia machine to operate normally.

Further, one end of the bypass branch 130 is connected to a common gas outlet or a fresh gas outlet of the anesthesia machine and the other end thereof is connected to the rear end of the inspiratory one-way valve in the inspiratory branch 111. It may be understood that both the common gas outlet and the fresh gas outlet may deliver the calibration gas. The calibration management unit controls the calibration gas to flow from the common gas outlet or the fresh gas outlet into the bypass branch 130, then pass through the bypass branch 130 into the inspiratory branch 111, and flow through the oxygen sensor 120 and the connection pipeline 116 into the expiratory branch 112, such that the automatic calibration of the oxygen sensor 120 is realized to ensure the reliability of the operation of the oxygen sensor 120 and to enable the anesthesia machine to operate normally.

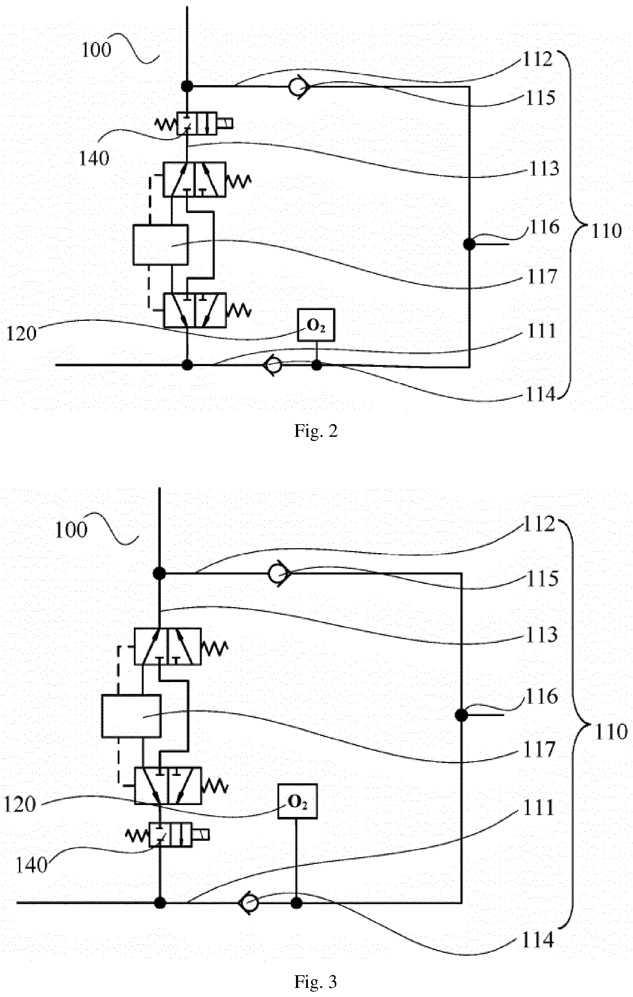

Referring to Figs. 2 and 3, in another embodiment of the present disclosure, the oxygen sensor calibration system 100 further includes a switch component 140. The switch component 140 is arranged in the absorption tank branch 113 and is used to control the opening and closing of the absorption tank branch 113. When the oxygen concentration calibration is performed, the switch component 140 closes the absorption tank branch 113 and the calibration gas may enter the inspiratory branch 111. Since the switch component 140 closes the absorption tank branch 113, when the calibration gas flows in the inspiratory branch 111, the calibration gas may not flow along the absorption tank branch 113 but may only continue to flow along the inspiratory branch 111, and may flow from the front end to the rear end of the inspiratory one-way valve 114 and flow through the oxygen sensor 120 and the connection pipeline 116 into the expiratory branch 112, such that the automatic calibration of the oxygen sensor 120 is realized to ensure the reliability of the operation of the oxygen sensor 120 and to enable the anesthesia machine to operate normally.

Preferably, the switch component 140 is an switch valve, an air-resistor, or another element capable of closing the absorption tank branch or preventing a large amount of gas from flowing through the absorption tank branch. In this way, all or most of the calibration gas may flow into the inspiratory branch 111 and pass through the connection line 116 and then flow out through the expiratory branch 112. Moreover, the switch component 140 may be arranged between the CO₂ absorption tank 117 and the inspiratory branch 111, as shown in Fig. 3, or may be also be arranged between the CO₂ absorption tank 117 and the expiratory branch 112, as shown in Fig. 2, as long as it may be ensured that the switch component 140 closes the absorption tank branch 113 or provides sufficient gas flow resistance in the absorption tank branch 113.

At this point, an input end of the inspiratory branch 111 may communicate with a gas source module of the anesthesia machine, or the common gas outlet, or the fresh gas outlet, and the calibration management unit controls the calibration gas to enter the inspiratory branch 111 during the oxygen concentration calibration. It may be understood that each of the gas source module, the common gas outlet and the fresh gas outlet may deliver the calibration gas. The calibration management unit controls the calibration gas to flow from the gas source module, the common gas outlet or the fresh gas outlet into the inspiratory branch 111, and flow through inspiratory one-way valve 114, the oxygen sensor 120 and the connection pipeline 116 into the expiratory branch 112, such that the automatic calibration of the oxygen sensor 120 is realized to ensure the reliability of the operation of the oxygen sensor 120 and to enable the anesthesia machine to operate normally.

Of course, in other embodiments of the present disclosure, the flow rate of the calibration gas may be adjusted by a flow meter or a control valve, etc., and then the calibration gas enters the bypass branch 130, and then enters the inspiratory branch 111 via the rear end of the inspiratory one-way valve 114. Alternatively, after the absorption tank branch 113 is closed by the switch component 140, the flow rate of the calibration gas is adjusted by the flow meter or the control valve, etc., and then the calibration gas enters the inspiratory branch 111. It should be noted that the communication between the various components of the calibration system for the oxygen sensor 120 is achieved through pipelines or by means of direct assembly and sealing. While the components described in some sections are directly connected to the inspiratory branch 111, the expiratory branch 112, etc., the components are actually connected to the inspiratory branch 111, the expiratory branch 112, etc. via the pipelines or by means of direct assembly and sealing, which are not described in detail here. Optionally, an inspiratory flow sensor is arranged in the inspiratory branch 111 for detecting the flow rate of the inspiratory gas in the inspiratory branch 111 to prevent a too large or too low flow rate of the inspiratory gas to ensure safe use. An expiratory flow sensor is arranged in the expiratory branch 112 for detecting the flow of the exhaled gas in the expiratory branch 112 to prevent a too large or too low flow rate of the exhaled gas to ensure safe use.

Referring to Fig. 4, the present disclosure further provides an anesthesia machine including an anesthetic supply device 200, an exhaust gas discharge device (not shown), and the oxygen sensor calibration system 100 as in the above embodiment. The gas inlet end of the inspiratory branch 111 of the breathing circuit 110 of the oxygen sensor calibration system 100 communicates with the anesthetic supply device 200, and the gas outlet end of the expiratory branch 112 of the breathing circuit 110 communicates with the exhaust gas discharge device via an expiratory valve. When the anesthesia machine is in use, the anesthetic supply device 200 supplies the inspiratory gas containing the anesthetic to the breathing circuit 110, the inspiratory gas enters the inspiratory branch 111 and is then supplied to the breathing end of the patient through the connection pipeline 116, and at the same time, the exhaled gas from the patient may also be delivered out of the breathing end of the patient. The exhaled gas is reused after CO₂ is absorbed by the CO₂ absorption tank 117 in the breathing circuit 110. The remaining exhaled gas is purified by the exhaust gas discharge device after passing through the expiratory valve 320. In this way, pollutions caused by direct discharge into the atmosphere may be prevented while affects on medical personnels may be prevented.

The anesthesia machine also has one or more of the gas source module, the common gas outlet, and the fresh gas outlet. When the oxygen sensor 120 is being calibrated, the calibration gas may enter the inspiratory branch 111 through the gas source module, the common gas outlet or the fresh gas outlet, and flow through the oxygen sensor 120 and the connection pipeline 116 into the expiratory branch 112, such that automatic calibration of the oxygen sensor 120 is realized to ensure the reliability of the operation of the oxygen sensor 120 and to enable the anesthesia machine to operate normally.

Further, the anesthesia machine further includes an expiratory device 300. The expiratory device 300 is arranged between the expiratory branch 112 and the exhaust gas discharge device for adjusting the pressure or flow rate of the exhaled gas. The expiratory device 300 includes a pressure adjustment structure and the expiratory valve 320. The gas inlet end and the gas outlet end of the expiratory valve 320 are respectively connected to an expiratory pipeline 310. One end of the expiratory valve 320 is connected to one end of the expiratory branch 112 through the expiratory pipeline 310, and the other end of the expiratory valve communicates with the exhaust gas discharge device through the expiratory pipeline 310. The expiratory valve 320 is used to discharge the exhaled gas from the patient, and the exhaled gas from the patient may enter the expiratory valve 320 through the expiratory branch 112 and through the expiratory pipeline 310, and is then discharged after being adjusted in pressure by the expiratory valve 320. A pressure control structure is used to adjust the flow rate or pressure of the exhaled gas delivered out of the expiratory valve 320 to adjust the valve closing pressure of the expiratory valve 320 to achieve the purpose of closing the expiratory valve 320 at a set pressure. In this way, if the inspiratory pressure in the inspiratory branch 111 of the breathing circuit 110 exceeds the valve closing pressure of the expiratory valve, the remaining inspiratory gas in the inspiratory branch 111 will directly flow into the expiratory valve 320 for release without reaching the patient, thereby ensuring that the pressure of the inspiratory gas at a patient end will not exceed the set pressure, so as to ensure the safety of the patient during use and improve the safety coefficient. The pressure control structure may be an adjustable pressure limitation valve (APL valve for short) or another structure capable of adjusting the pressure of the exhaled gas. In this embodiment, the pressure control structure includes an adjustment pipeline 340 for inputting and outputting an adjustment gas and an adjustment valve 330 arranged in the adjustment pipeline 340. An output end of the adjustment valve 330 communicates with the expiratory valve 320. The adjustment valve 330 may adjust the pressure or flow rate of the pressure adjustment gas in the adjustment pipeline 340 to achieve the pressure adjustment of the exhaled gas in the expiratory valve 320.

With the advancement of science and technology, many self-test items of the anesthesia machine, such as gas tightness, flow measurement accuracy, and flow sensor calibration, have been fully automated, which not only frees up the medical personnels to enable them to focus more on patients and surgical procedures, but also improves the reliability of equipment and avoids deviations caused by manual intervention.

Further, the anesthesia machine further includes a control unit, which controls the oxygen sensor calibration system to perform the oxygen sensor calibration during the self-test of the anesthesia machine. The self-test of the anesthesia machine includes one or more of gas tightness detection, backup flow control system detection or flow sensor calibration, etc. The control unit may control the anesthesia machine to carry out the self-test when being started up and in standby, or the anesthesia machine may also carry out the self-test regularly or according to a control signal input by a user.

The calibration of the oxygen sensor is completed together with the self-test of the anesthesia machine, which does not require operator intervention or concern at all, thereby reducing the operator's burden and preventing potential safety hazards to the patient due to inaccurate measurement of the oxygen concentration during use of the anesthesia machine caused by the operator forgetting to perform the calibration. Further, completing the calibration of the oxygen sensor and the self-test for the air tightness of the anesthesia machine together may also eliminate the operation of closing the connection pipeline by the operator.

The present disclosure further provides a calibration method of an oxygen sensor calibration system. The calibration method is applied to the above-mentioned oxygen sensor calibration system 100. The calibration method includes the following steps:
controlling the calibration gas to enter the inspiratory branch 111 and flow out through the oxygen sensor 120, the connection pipeline 116 and the expiratory branch 112; and
performing the oxygen concentration calibration according to the calibration gas flowing through the oxygen sensor 120.

When the oxygen sensor 120 is being calibrated, the calibration management unit controls the calibration gas to enter the inspiratory branch 111 and flow out through the oxygen sensor 120, the connection pipeline 116, and the expiratory branch 112 without closing the connection pipeline 116. The calibration management unit performs the oxygen concentration calibration according to the calibration gas flowing through the oxygen sensor 120 to realize the automatic calibration of the oxygen sensor 120. Moreover, when the calibration gas flows through the oxygen sensor 120, the oxygen sensor 120 may output a corresponding value of the current according to the value of the actual oxygen concentration in the calibration gas, and the control unit stores the function relation between the values of the oxygen concentration and the current to realize the calibration operation of the oxygen sensor 120.

The various technical features of the embodiments described above can be arbitrarily combined. In order to simplify the description, all possible combinations of the various technical features in the above embodiments have not been described. However, any combination of these technical features should be considered to fall within the scope of the disclosure of this description as long as there is no contradiction.

The above-mentioned embodiments merely represent several implementations of the present disclosure, giving specifics and details thereof, but should not be understood as limiting the disclosure thereby. It should be noted that a person of ordinary skill in the art could also make several variations and improvements without departing from the present disclosure. These variations and improvements all fall within the scope of protection of the present disclosure. The scope of protection of the present invention is limited by the appended claims only.

## Claims

1. An anesthesia machine including an oxygen sensor calibration system (100), the oxygen sensor calibration system (100) comprising:
a breathing circuit (110) comprising an inspiratory branch (111), an expiratory branch (112), an absorption tank branch (113), an inspiratory one-way valve (114), a connection pipeline (116) and an expiratory one-way valve (115), wherein the inspiratory branch (111) and the expiratory branch (112) communicate via the connection pipeline (116), the inspiratory one-way valve (114) is arranged in the inspiratory branch (111), the expiratory one-way valve (115) is arranged in the expiratory branch (112), one end of the absorption tank branch (113) communicates with the inspiratory branch (111) and is located at a front end of the inspiratory one-way valve (114), and the other end of the absorption tank branch (113) communicates with the expiratory branch (112) and is located at a rear end of the expiratory one-way valve (115); and
an oxygen sensor (120) connected to the inspiratory branch (111), with a joint being located at a rear end of the inspiratory one-way valve (114);
wherein each of the inspiratory and expiratory one-way valves (114, 115) is configured to allow flow from the front end thereof to the rear end thereof;
wherein the anesthesia machine further includes an anesthetic supply device and an exhaust gas discharge device;
wherein one end of the inspiratory branch (111) of the breathing circuit (110) of the oxygen sensor (120) calibration system communicates with the anesthetic supply device, and one end of the expiratory branch (112) of the breathing circuit (110) communicates with the exhaust gas discharge device;
wherein the anesthesia machine is configured such that, during use of the anesthesia machine, the anesthetic supply device supplies an inspiratory gas containing an anesthetic to the breathing circuit (110), the inspiratory gas enters the inspiratory branch (111) and is then supplied to a patient through the connection pipeline (116), and at the same time, an exhaled gas from the patient also reaches the expiratory branch (112) through the connection pipeline (116) of the breathing circuit (110);
wherein the anesthesia machine is provided with one or more of a gas source module, a common gas outlet and a fresh gas outlet, and when the oxygen sensor (120) is being calibrated, the gas source module, the common gas outlet or the fresh gas outlet supplies the calibration gas to the inspiratory branch (111);
the oxygen sensor calibration system further comprising a calibration management unit, the calibration management unit being configured to control a calibration gas to enter the inspiratory branch (111) and flow out through the oxygen sensor (120), the connection pipeline (116) and the expiratory branch (112), and the calibration management unit being configured to perform an oxygen concentration calibration according to the calibration gas flowing through the oxygen sensor (120);
the oxygen sensor calibration system further comprising a bypass branch (130), wherein
the bypass branch (130) and the inspiratory branch (111) are connected between the inspiratory one-way valve (114) and the oxygen sensor (120);
the calibration management unit being configured to control the calibration gas to enter the inspiratory branch (111) through the bypass branch (130) during the oxygen concentration calibration; and
wherein one end of the bypass branch (130) is connected to the common gas outlet or the fresh gas outlet of the anesthesia machine, and the other end thereof is connected to the rear end of the inspiratory one-way valve (114) in the inspiratory branch (111).

2. An anesthesia machine including an oxygen sensor calibration system (100),
the oxygen sensor calibration system (100) comprising: a breathing circuit (110) comprising an inspiratory branch (111), an expiratory branch (112), an absorption tank branch (113), an inspiratory one-way valve (114), a connection pipeline (116) and an expiratory one-way valve (115), wherein the inspiratory branch (111) and the expiratory branch (112) communicate via the connection pipeline (116), the inspiratory one-way valve (114) is arranged in the inspiratory branch (111), the expiratory one-way valve (115) is arranged in the expiratory branch (112), one end of the absorption tank branch (113) communicates with the inspiratory branch (111) and is located at a front end of the inspiratory one-way valve (114), and the other end of the absorption tank branch (113) communicates with the expiratory branch (112) and is located at a rear end of the expiratory one-way valve (115); and an oxygen sensor (120) connected to the inspiratory branch (111), with a joint being located at a rear end of the inspiratory one-way valve (114);
wherein each of the inspiratory and expiratory one-way valves (114, 115) is configured to allow flow from the front end thereof to the rear end thereof;
wherein the anesthesia machine further includes an anesthetic supply device and an exhaust gas discharge device;
wherein one end of the inspiratory branch (111) of the breathing circuit (110) of the oxygen sensor (120) calibration system communicates with the anesthetic supply device, and one end of the expiratory branch (112) of the breathing circuit (110) communicates with the exhaust gas discharge device;
wherein the anesthesia machine is configured such that, during use of the anesthesia machine, the anesthetic supply device supplies an inspiratory gas containing an anesthetic to the breathing circuit (110), the inspiratory gas enters the inspiratory branch (111) and is then supplied to a patient through the connection pipeline (116), and at the same time, an exhaled gas from the patient also reaches the expiratory branch (112) through the connection pipeline (116) of the breathing circuit (110);
wherein the anesthesia machine is provided with one or more of a gas source module,
a common gas outlet and a fresh gas outlet, and when the oxygen sensor (120) is being calibrated, the gas source module, the common gas outlet or the fresh gas outlet supplies the calibration gas to the inspiratory branch (111);
the oxygen sensor calibration system further comprising a calibration management unit,
the calibration management unit being configured to control a calibration gas to enter the inspiratory branch (111) and flow out through the oxygen sensor (120), the connection pipeline (116) and the expiratory branch (112), and
the calibration management unit being configured to perform an oxygen concentration calibration according to the calibration gas flowing through the oxygen sensor (120);
the calibration management unit further comprising a switch component (140),
wherein the switch component (140) is arranged in the absorption tank branch (113) so as to be configured to control the opening and closing of the absorption tank branch (113); and
wherein the calibration management unit is configured such that, when the oxygen concentration calibration is performed, the switch component (140) closes the absorption tank branch (113) such that the calibration gas is capable of entering the inspiratory branch (111); and
wherein an input end of the inspiratory branch (111) communicates with the gas source module of the anesthesia machine, or the common gas outlet, or the fresh gas outlet, and the calibration management unit being configured to control the calibration gas to enter the inspiratory branch (111) during the oxygen concentration calibration.

3. The anesthesia machine of claim 2, wherein the switch component (140) is a switch valve or an air-resistor.

4. The anesthesia machine according to any one of claims 1-3, wherein the oxygen sensor (120) is a chemical oxygen sensor, and the calibration management unit is configured to calibrate the oxygen sensor (120) with the calibration gas having at least two different oxygen concentrations; or
the oxygen sensor (120) is a paramagnetic oxygen sensor, and the calibration management unit is configured to calibrate the oxygen sensor (120) with the calibration gas having at least one oxygen concentration.

5. The anesthesia machine of claim 1, further comprising an expiratory device, which is arranged between the expiratory branch (112) and the exhaust gas discharge device for adjusting the flow rate or pressure of the exhaled gas.

6. The anesthesia machine of claim 1, further comprising a control unit, which is configured to control the oxygen sensor (120) calibration system to perform the oxygen sensor calibration during a self-test of the anesthesia machine.

7. The anesthesia machine of claim 6, wherein the self-test of the anesthesia machine further comprises one or more of gas tightness detection, flow control system test or flow sensor calibration.

8. A method of calibrating the oxygen sensor (120) of an anesthesia machine according to any one of claims 1-7 with the oxygen sensor calibration system (100) of the anesthesia machine,
the calibration method comprising the following steps: controlling, by the calibration management unit of the oxygen sensor calibration system (100),
the calibration gas to enter the inspiratory branch (111) of the oxygen sensor calibration system (100),
and flow out through the oxygen sensor (120), the connection pipeline (116) and the expiratory branch (112) of the oxygen sensor calibration system (100); and
performing, by the calibration management unit of the oxygen sensor calibration system (100),
an oxygen concentration calibration of the oxygen sensor (120) according to the calibration gas flowing through the oxygen sensor (120).

9. The method of claim 8, wherein the calibration method is performed during a self-test of the anesthesia machine.

10. The method of claim 8, wherein the anesthesia machine is according to claim 1; and the step of controlling the calibration gas to enter the inspiratory branch (111) comprises: controlling the calibration gas to enter the inspiratory branch (111) through the bypass branch (130) of the oxygen sensor calibration system (100).

## Patentansprüche

1. Anästhesiegerät mit einem Kalibriersystem für einen Sauerstoffsensor (100), wobei das Sauerstoffsensorkalibriersystem (100) Folgendes umfasst:
ein Atemkreislaufsystem (110), das einen Inspirationszweig (111), einen Exspirationszweig (112), einen Absorptionstankzweig (113), ein inspiratorisches Rückschlagventil (114), eine Verbindungsleitung (116) und ein exspiratorisches Rückschlagventil (115) umfasst, wobei der Inspirationszweig (111) und der Exspirationszweig (112) über die Verbindungsleitung (116) miteinander kommunizieren, das inspiratorische Rückschlagventil (114) im Inspirationszweig (111) angeordnet ist, das exspiratorische Rückschlagventil (115) im Exspirationszweig (112) angeordnet ist, ein Ende des Absorptionstankzweigs (113) mit dem Inspirationszweig (111) kommuniziert und sich an einem vorderen Ende des inspiratorischen Rückschlagventils (114) befindet, und das andere Ende des Absorptionstankzweigs (113) mit dem Exspirationszweig (112) kommuniziert und sich an einem hinteren Ende des exspiratorischen Rückschlagventils (115) befindet;
ein Sauerstoffsensor (120), der mit dem Inspirationszweig (111) verbunden ist, wobei eine Verbindung am hinteren Ende des inspiratorischen Rückschlagventils (114) angeordnet ist;
wobei jedes der inspiratorischen und exspiratorischen Rückschlagventile (114, 115) so konfiguriert ist, dass es einen Fluss vom vorderen Ende zum hinteren Ende desselben ermöglicht;
wobei das Anästhesiegerät femer eine Anästhetikum-Zufuhrvorrichtung und eine Vorrichtung zur Ableitung von Abgasen umfasst;
wobei ein Ende des Inspirationszweigs (111) des Atemkreislaufs (110) des Kalibriersystems für den Sauerstoffsensor (120) mit der Anästhetikum-Zufuhrvorrichtung kommuniziert und ein Ende des Exspirationszweigs (112) des Atemkreislaufs (110) mit der Vorrichtung zur Ableitung von Abgasen kommuniziert;
wobei das Anästhesiegerät so konfiguriert ist, dass während des Gebrauchs des Anästhesiegeräts die Anästhetikum-Zufuhrvorrichtung ein inspiratorisches Gas, das ein Anästhetikum enthält, in den Atemkreislauf (110) einspeist, das inspiratorische Gas in den Inspirationszweig (111) eintritt und dann über die Verbindungsleitung (116) einem Patienten zugeführt wird, und gleichzeitig ein vom Patienten ausgeatmetes Gas ebenfalls über die Verbindungsleitung (116) des Atemkreislaufs (110) den Exspirationszweig (112) erreicht;
Wobei das Anästhesiegerät mit einem oder mehreren der folgenden Elemente versehen ist: Einem Gasquellenmodul, einem gemeinsamen Gasauslass und einem Frischgasauslass, und wobei beim Kalibrieren des Sauerstoffsensors (120) das Gasquellenmodul, der gemeinsame Gasauslass oder der Frischgasauslass das Kalibriergas in den Inspirationszweig einspeist;
wobei das Sauerstoffsensor-Kalibriersystem femer eine Kalibrierungsverwaltungseinheit umfasst, wobei die Kalibrierungsverwaltungseinheit so konfiguriert ist, dass sie ein Kalibriergas in den Inspirationszweig (111) eintreten lässt und durch den Sauerstoffsensor (120), die Verbindungsleitung (116) und den Exspirationszweig (112) austreten lässt, und wobei die Kalibrierungsverwaltungseinheit so konfiguriert ist, dass sie eine Sauerstoffkonzentrationskalibrierung basierend auf dem durch den Sauerstoffsensor (120) strömenden Kalibriergas durchführt;
das Sauerstoffsensor-Kalibriersystem umfasst femer einen Bypass-Zweig (130), wobei der Bypass-Zweig (130) und der Inspirationszweig (111) zwischen dem inspiratorischen Rückschlagventil (114) und dem Sauerstoffsensor (120) verbunden sind;
wobei die Kalibrierungsverwaltungseinheit so konfiguriert ist, dass sie das Kalibriergas während der Sauerstoffkonzentrationskalibrierung durch den Bypass-Zweig (130) in den Inspirationszweig (111) eintreten lässt.
Wobei ein Ende des Bypass-Zweigs (130) mit dem gemeinsamen Gasauslass oder dem Frischgasauslass des Anästhesiegeräts verbunden ist, und dessen anderes Ende mit dem hinteren Ende des inspiratorischen Rückschlagventils (114) im Inspirationszweig verbunden ist.

2. Anästhesiegerät mit einem Sauerstoffsensorkalibriersystem (100), wobei das Sauerstoffsensorkalibriersystem (100) folgendes umfasst: Ein Atemkreislaufsystem (110), das einen Inspirationszweig (111), einen Exspirationszweig (112), einen Absorptionstankzweig (113), ein inspiratorisches Rückschlagventil (114), eine Verbindungspipeline (116) und ein exspiratorisches Rückschlagventil (115) umfasst, wobei der Inspirationszweig (111) und der Exspirationszweig (112) über die Verbindungspipeline (116) miteinander verbunden sind, wobei das inspiratorische Rückschlagventil (114) im Inspirationszweig (111) angeordnet ist, wobei das exspiratorische Rückschlagventil (115) im Exspirationszweig (112) angeordnet ist, wobei ein Ende des Absorptionstankzweigs (113) mit dem Inspirationszweig (111) verbunden ist und sich am vorderen Ende des inspiratorischen Rückschlagventils (114) befindet, und das andere Ende des Absorptionstankzweigs (113) mit dem Exspirationszweig (112) verbunden ist und sich am hinteren Ende des exspiratorischen Rückschlagventils (115) befindet; und ein Sauerstoffsensor (120), der mit dem Inspirationszweig (111) verbunden ist, wobei die Anschlussstelle am hinteren Ende des inspiratorischen Rückschlagventils (114) angeordnet ist;
wobei jedes der inspiratorischen und exspiratorischen Rückschlagventile (114, 115) so konfiguriert ist, dass es einen Fluss vom vorderen Ende zum hinteren Ende erlaubt;
wobei das Anästhesiegerät weiterhin ein Anästhesie Versorgungsgerät und eine Abluftvorrichtung umfasst;
wobei das Anästhesiegerät derart konfiguriert ist, dass während des Gebrauchs des Anästhesiegeräts das Anästhesie Versorgungsgerät ein Inspirationsgas, das ein Anästhetikum enthält, dem Atemkreislaufsystem (110) zuführt, das Inspirationsgas in den Inspirationszweig (111) eintritt und anschließend über die Verbindungspipeline (116) einem Patienten zugeführt wird, und gleichzeitig ein vom Patienten ausgeatmetes Gas ebenfalls über die Verbindungspipeline (116) des Atemkreislaufsystems (110) den Exspirationszweig (112) erreicht;
wobei das Anästhesiegerät mit einem oder mehreren der folgenden Elemente versehen ist: Einem Gasquellenmodul, einem gemeinsamen Gasauslass und einem Frischgasauslass, und wenn der Sauerstoffsensor (120) kalibriert wird, das Gasquellenmodul, der gemeinsame Gasauslass oder der Frischgasauslass das Kalibriergas dem Inspirationszweig (111) zuführt; wobei das Kalibriersystem für den Sauerstoffsensor ferner eine Kalibriermanagementeinheit umfasst, wobei die Kalibriermanagementeinheit so konfiguriert ist, dass sie ein Kalibriergas in den Inspirationszweig (111) einleitet und dieses durch den Sauerstoffsensor (120), die Verbindungspipeline (116) und den Exspirationszweig (112) ausströmen lässt,
und die Kalibriermanagementeinheit konfiguriert ist, eine Sauerstoffkonzentrationskalibrierung anhand des durch den Sauerstoffsensor (120) strömenden Kalibriergases durchzuführen. Wobei ein Ende des Inspirationszweigs (111) des Atemkreislaufsystems (110) des Kalibriersystems für den Sauerstoffsensor (120) mit dem Anästhesie Versorgungsgerät verbunden ist, und ein Ende des Exspirationszweigs (112) des Atemkreislaufsystems (110) mit der Abluftvorrichtung verbunden ist.
l'unité de gestion d'etalonnage etant configuree pour realiser un etalonnage de concentration d'oxygène selon le gaz d'etalonnage s'ecoulant a travers le capteur d'oxygène (120) ;
Wobei die Kalibriermanagementeinheit femer eine Schaltkomponente (140) umfasst, wobei die Schaltkomponente (140) im Absorptionstankzweig (113) angeordnet ist, um die Öffnung und Schließung des Absorptionstankzweigs (113) zu steuern;
und wobei die Kalibriermanagementeinheit so konfiguriert ist, dass beim Durchführen der Sauerstoffkonzentrationskalibrierung die Schaltkomponente (140) den Absorptionstankzweig (113) schließt, so dass das Kalibriergas in der Lage ist, in den Inspirationszweig (111) einzutreten;
und wobei ein Eingangsende des Inspirationszweigs (111) mit dem Gasquellenmodul des Anästhesiegeräts oder dem gemeinsamen Gasauslass oder dem Frischgasauslass in Verbindung steht, und die Kalibriermanagementeinheit konfiguriert ist, das Kalibriergas während der Sauerstoffkonzentrationskalibrierung in den Inspirationszweig (111) einzuleiten.

3. Die Anästhesiemaschine nach Anspruch 2, wobei die Schaltkomponente (140) ein Schaltventil oder ein Luftwiderstand ist.

4. Die Anästhesiemaschine nach einem der Ansprüche 1 bis 3, wobei der Sauerstoffsensor (120) ein chemischer Sauerstoffsensor ist,
und die Kalibriermanagementeinheit so konfiguriert ist, den Sauerstoffsensor (120) mit einem Kalibriergas mit mindestens zwei verschiedenen Sauerstoffkonzentrationen zu kalibrieren; oder der Sauerstoffsensor (120) ist ein paramagnetischer Sauerstoffsensor, und die Kalibriermanagementeinheit ist so konfiguriert, den Sauerstoffsensor (120) mit einem Kalibriergas mit mindestens einer Sauerstoffkonzentration zu kalibrieren.

5. Die Anästhesiemaschine nach Anspruch 1, weiterhin umfassend eine Ausatmungsvorrichtung, die zwischen dem Ausatmungszweig (112) und der Ausatemgas-Entsorgungsvorrichtung angeordnet ist, um die Durchflussrate oder den Druck des ausgeatmeten Gases zu regeln.

6. Die Anästhesiemaschine nach Anspruch 1, weiterhin umfassend eine Steuereinheit, die so konfiguriert ist, das Kalibriersystem des Sauerstoffsensors (120) während eines Selbsttests der Anästhesiemaschine zur Durchführung der Sauerstoffsensorkalibrierung zu steuem.

7. Die Anästhesiemaschine nach Anspruch 6, wobei der Selbsttest der Anästhesiemaschine weiterhin eine oder mehrere der folgenden Prüfungen umfasst: Dichtigkeitsprüfung, Test des Durchflussregelsystems oder Kalibrierung des Durchflusssensors.

8. Ein Verfahren zur Kalibrierung des Sauerstoffsensors (120) einer Anästhesiemaschine gemäß einem der Ansprüche 1 bis 7 mit dem Sauerstoffsensorkalibriersystem (100) der Anästhesiemaschine, wobei das Kalibrierverfahren folgende Schritte umfasst:
Steuern des Kalibriergases durch die Kalibrierverwaltungseinheit des Sauerstoffsensorkalibriersystems (100), sodass das Kalibriergas in den Inspirationszweig (111) des Sauerstoffsensorkalibriersystems (100) eintritt und durch den Sauerstoffsensor (120), die Verbindungsleitung (116) und den Exspirationszweig (112) des Sauerstoffsensorkalibriersystems (100) austritt; und
Durchführen einer Sauerstoffkonzentrationskalibrierung des Sauerstoffsensors (120) durch die Kalibrierverwaltungseinheit des Sauerstoffsensorkalibriersystems (100) basierend auf dem durch den Sauerstoffsensor (120) strömenden Kalibriergas.

9. Verfahren nach Anspruch 8, wobei das Kalibrierverfahren während eines Selbsttests der Anästhesiemaschine durchgeführt wird

10. Verfahren nach Anspruch 8, wobei die Anästhesiemaschine gemäß Anspruch 1 ist;
Verfahren nach Anspruch 8, wobei die Anästhesiemaschine gemäß Anspruch 1 ist; und der Schritt des Steuerns des Kalibriergases zum Eintritt in den Inspirationszweig (111) umfasst: Steuern des Kalibriergases zum Eintritt in den Inspirationszweig (111) über den Bypasszweig (130) des Sauerstoffsensorkalibriersystems (100).

## Revendications

1. Machine d'anesthésie incluant un système d'étalonnage de capteur d'oxygène (100), le système d'étalonnage de capteur d'oxygène (100) comprenant :
un circuit de respiration (110) comprenant une branche inspiratoire (111), une branche expiratoire (112), une branche de réservoir d'absorption (113), un clapet anti-retour inspiratoire (114), une conduite de liaison (116) et un clapet anti-retour expiratoire (115), où la branche inspiratoire (111) et la branche expiratoire (112) communiquent via la conduite de liaison (116), le clapet anti-retour inspiratoire (114) est agencé dans la branche inspiratoire (111), le clapet anti-retour expiratoire (115) est agencé dans la branche expiratoire (112), une première extrémité de la branche de réservoir d'absorption (113) communique avec la branche inspiratoire (111) et est située au niveau d'une extrémité avant du clapet anti-retour inspiratoire (114), et l'autre extrémité de la branche de réservoir d'absorption (113) communique avec la branche expiratoire (112) et est située au niveau d'une extrémité arrière du clapet anti-retour expiratoire (115) ; et
un capteur d'oxygène (120) relié à la branche inspiratoire (111), avec un raccord qui est situé au niveau d'une extrémité arrière du clapet anti-retour inspiratoire (114) ;
où chacun des clapets anti-retour inspiratoire et expiratoire (114, 115) est configuré pour permettre un écoulement de l'extrémité avant de ceux-ci vers l'extrémité arrière de ceux-ci ;
où la machine d'anesthésie inclut en outre un dispositif d'alimentation en anesthésique et un dispositif de libération de gaz d'évacuation ;
où
une première extrémité de la branche inspiratoire (111) du circuit de respiration (110) du système d'étalonnage du capteur d'oxygène (120) communique avec le dispositif d'alimentation en anesthésique, et une première extrémité de la branche expiratoire (112) du circuit de respiration (110) communique avec le dispositif de libération de gaz d'évacuation ;
où la machine d'anesthésie est configurée de telle sorte que, durant une utilisation de la machine d'anesthésie, le dispositif d'alimentation en anesthésique fournit un gaz inspiratoire contenant un anesthésique au circuit de respiration (110), le gaz inspiratoire entre dans la branche inspiratoire (111) et est ensuite fourni à un patient par le biais de la conduite de liaison (116), et en même temps, un gaz exhalé du patient atteint également la branche expiratoire (112) par le biais de la conduite de liaison (116) du circuit de respiration (110) ;
où
la machine d'anesthésie est dotée d'un ou plusieurs éléments parmi un module de source de gaz, une sortie de gaz commune et une sortie de gaz frais, et lorsque le capteur d'oxygène (120) est en train d'être étalonné, le module de source de gaz, la sortie de gaz commune ou la sortie de gaz frais fournit le gaz d'étalonnage à la branche inspiratoire (111) ;
le système d'étalonnage de capteur d'oxygène comprenant en outre une unité de gestion d'étalonnage, l'unité de gestion d'étalonnage étant configurée pour amener un gaz d'étalonnage à entrer dans la branche inspiratoire (111) et à sortir en passant par le capteur d'oxygène (120), la conduite de liaison (116) et la branche expiratoire (112), et l'unité de gestion d'étalonnage étant configurée pour réaliser un étalonnage de concentration d'oxygène selon le gaz d'étalonnage s'écoulant à travers le capteur d'oxygène (120) ;
le système d'étalonnage de capteur d'oxygène comprenant en outre une branche de dérivation (130), où la branche de dérivation (130) et la branche inspiratoire (111) sont reliées entre le clapet anti-retour inspiratoire (114) et le capteur d'oxygène (120) ;
l'unité de gestion d'étalonnage étant configurée pour amener le gaz d'étalonnage à entrer dans la branche inspiratoire (111) par la branche de dérivation (130) durant l'étalonnage de concentration d'oxygène ; et
où une première extrémité de la branche de dérivation (130) est reliée à la sortie de gaz commune ou à la sortie de gaz frais de la machine d'anesthésie, et l'autre extrémité de celle-ci est reliée à l'extrémité arrière du clapet anti-retour inspiratoire (114) dans la branche inspiratoire (111).

2. Machine d'anesthésie incluant un système d'étalonnage de capteur d'oxygène (100), le système d'étalonnage de capteur d'oxygène (100) comprenant : un circuit de respiration (110) comprenant une branche inspiratoire (111), une branche expiratoire (112), une branche de réservoir d'absorption (113), un clapet anti-retour inspiratoire (114), une conduite de liaison (116) et un clapet anti-retour expiratoire (115), où la branche inspiratoire (111) et la branche expiratoire (112) communiquent via la conduite de liaison (116), le clapet anti-retour inspiratoire (114) est agencé dans la branche inspiratoire (111), le clapet anti-retour expiratoire (115) est agencé dans la branche expiratoire (112), une première extrémité de la branche de réservoir d'absorption (113) communique avec la branche inspiratoire (111) et est située au niveau d'une extrémité avant du clapet anti-retour inspiratoire (114), et l'autre extrémité de la branche de réservoir d'absorption (113) communique avec la branche expiratoire (112) et est située au niveau d'une extrémité arrière du clapet anti-retour expiratoire (115) ; et un capteur d'oxygène (120) relié à la branche inspiratoire (111), avec un raccord qui est situé au niveau d'une extrémité arrière du clapet anti-retour inspiratoire (114) ;
où chacun des clapets anti-retour inspiratoire et expiratoire (114, 115) est configuré pour permettre un écoulement de l'extrémité avant de ceux-ci vers l'extrémité arrière de ceux-ci ;
où la machine d'anesthésie inclut en outre un dispositif d'alimentation en anesthésique et un dispositif de libération de gaz d'évacuation ;
où une première extrémité de la branche inspiratoire (111) du circuit de respiration (110) du système d'étalonnage du capteur d'oxygène (120) communique avec le dispositif d'alimentation en anesthésique, et une première extrémité de la branche expiratoire (112) du circuit de respiration (110) communique avec le dispositif de libération de gaz d'évacuation ;
où la machine d'anesthésie est configurée de telle sorte que, durant une utilisation de la machine d'anesthésie, le dispositif d'alimentation en anesthésique fournit un gaz inspiratoire contenant un anesthésique au circuit de respiration (110), le gaz inspiratoire entre dans la branche inspiratoire (111) et est ensuite fourni à un patient par le biais de la conduite de liaison (116), et en même temps, un gaz exhalé du patient atteint également la branche expiratoire (112) par le biais de la conduite de liaison (116) du circuit de respiration (110) ;
où la machine d'anesthésie est dotée d'un ou plusieurs éléments parmi un module de source de gaz, une sortie de gaz commune et une sortie de gaz frais, et lorsque le capteur d'oxygène (120) est en train d'être étalonné, le module de source de gaz, la sortie de gaz commune ou la sortie de gaz frais fournit le gaz d'étalonnage à la branche inspiratoire (111) ;
le système d'étalonnage de capteur d'oxygène comprenant en outre une unité de gestion d'étalonnage, l'unité de gestion d'étalonnage étant configurée pour amener un gaz d'étalonnage à entrer dans la branche inspiratoire (111) et à sortir en passant par le capteur d'oxygène (120), la conduite de liaison (116) et la branche expiratoire (112), et
l'unité de gestion d'étalonnage étant configurée pour réaliser un étalonnage de concentration d'oxygène selon le gaz d'étalonnage s'écoulant à travers le capteur d'oxygène (120) ;
l'unité de gestion d'étalonnage comprenant en outre un composant de commutation (140), où le composant de commutation (140) est agencé dans la branche de réservoir d'absorption (113) de sorte à être configuré pour commander l'ouverture et la fermeture de la branche de réservoir d'absorption (113) ; et
où l'unité de gestion d'étalonnage est configurée de telle sorte que, lorsque l'étalonnage de concentration d'oxygène est réalisé, le composant de commutation (140) ferme la branche de réservoir d'absorption (113) de telle sorte que le gaz d'étalonnage est en mesure d'entrer dans la branche inspiratoire (111) ; et
où une extrémité d'entrée de la branche inspiratoire (111) communique avec le module de source de gaz de la machine d'anesthésie, ou la sortie de gaz commune, ou la sortie de gaz frais, et l'unité de gestion d'étalonnage étant configurée pour amener le gaz d'étalonnage à entrer dans la branche inspiratoire (111) durant l'étalonnage de concentration d'oxygène.

3. Machine d'anesthésie selon la revendication 2, dans laquelle le composant de commutation (140) est une vanne de commutation ou une résistance à air.

4. Machine d'anesthésie selon l'une quelconque des revendications 1 à 3, dans laquelle le capteur d'oxygène (120) est un capteur d'oxygène chimique, et l'unité de gestion d'étalonnage est configurée pour étalonner le capteur d'oxygène (120) avec le gaz d'étalonnage avec au moins deux concentrations d'oxygène différentes ; ou
le capteur d'oxygène (120) est un capteur d'oxygène paramagnétique, et l'unité de gestion d'étalonnage est configurée pour étalonner le capteur d'oxygène (120) avec le gaz d'étalonnage avec au moins une concentration d'oxygène.

5. Machine d'anesthésie selon la revendication 1, comprenant en outre un dispositif expiratoire, lequel est agencé entre la branche expiratoire (112) et le dispositif de libération de gaz d'évacuation afin de régler le débit ou la pression du gaz exhalé.

6. Machine d'anesthésie selon la revendication 1, comprenant en outre une unité de commande, laquelle est configurée pour amener le système d'étalonnage du capteur d'oxygène (120) à réaliser l'étalonnage du capteur d'oxygène durant un autotest de la machine d'anesthésie.

7. Machine d'anesthésie selon la revendication 6, où l'autotest de la machine d'anesthésie comprend en outre un ou plusieurs contrôles parmi une détection d'étanchéité au gaz, un test du système de commande d'écoulement ou un étalonnage de capteur d'écoulement.

8. Procédé d'étalonnage du capteur d'oxygène (120) d'une machine d'anesthésie selon l'une quelconque des revendications 1 à 7 avec le système d'étalonnage de capteur d'oxygène (100) de la machine d'anesthésie, le procédé d'étalonnage comprenant les étapes suivantes consistant à :
amener, par le bais de l'unité de gestion d'étalonnage du système d'étalonnage de capteur d'oxygène (100), le gaz d'étalonnage à entrer dans la branche inspiratoire (111) du système d'étalonnage de capteur d'oxygène (100) et à sortir par le capteur d'oxygène (120), la conduite de liaison (116) et la branche expiratoire (112) du système d'étalonnage de capteur d'oxygène (100) ;
et
réaliser, par le biais de l'unité de gestion d'étalonnage du système d'étalonnage de capteur d'oxygène (100), un étalonnage de concentration d'oxygène du capteur d'oxygène (120) selon le gaz d'étalonnage s'écoulant à travers le capteur d'oxygène (120).

9. Procédé selon la revendication 8, où le procédé d'étalonnage est réalisé durant un autotest de la machine d'anesthésie.

10. Procédé selon la revendication 8, où la machine d'anesthésie est selon la revendication 1 ;
et l'étape consistant à amener le gaz d'étalonnage à entrer dans la branche inspiratoire (111) comprend : le fait d'amener le gaz d'étalonnage à entrer dans la branche inspiratoire (111) par le biais de la branche de dérivation (130) du système d'étalonnage de capteur d'oxygène (100).
